# EUROPEAN PATENT APPLICATION

(11) **EP 4 338 675 A1**
(43) Date of publication of application: **20.03.2024**
(21) Application number: 22807446.4
(22) Date of filing: 09.05.2022
(51) Int. Cl.: A61B 6/00

(54) **INFORMATION PROCESSING DEVICE, DIAGNOSIS ASSISTANCE PROCESSING DEVICE, INFORMATION PROCESSING METHOD, DIAGNOSIS ASSISTANCE PROCESSING METHOD, INFORMATION PROCESSING PROGRAM, AND DIAGNOSIS ASSISTANCE PROCESSING PROGRAM**

(30) Priority: 11.05.2021 JP 2021080524
(71) Applicant: FUJIFILM Corporation, Tokyo 106-8620 (JP)
(72) Inventor: HAYASHI, Hiromu, Ashigarakami-gun, Kanagawa 258-8538 (JP); TANINAI, Koji, Ashigarakami-gun, Kanagawa 258-8538 (JP); MAKINO, Kazuhiro, Ashigarakami-gun, Kanagawa 258-8538 (JP); KOBAYASHI, Takeyasu, Ashigarakami-gun, Kanagawa 258-8538 (JP); BETTOYASHIKI, Akihito, Ashigarakami-gun, Kanagawa 258-8538 (JP)
(74) Representative: Hughes, Andrea Michelle
(86) International application number: PCT/JP2022/019707
(87) International publication number: WO 2022/239745

(57) **Abstract**

An information processing apparatus includes at least one processor, in which the processor is configured to perform diagnosis support processing on one medical image and derive a plurality of diagnosis support processing results, present selection information for allowing a user to select at least one diagnosis support processing result among the plurality of derived diagnosis support processing results, and output a diagnosis support processing result selected by the user according to the selection information.

## Description

### Technical Field

The present disclosure relates to an information processing apparatus, a diagnosis support processing device, an information processing method, a diagnosis support processing method, an information processing program, and a diagnosis support processing program.

### Background Art

The technology is known which performs diagnosis support processing for medical images such as radiation images. For example, JP 2019-093137 A describes a technology that applies a trained learning network to a captured image and notifies a user in a case in which clinical findings are observed.

### SUMMARY OF THE INVENTION

### Technical Problem

Incidentally, in some cases, a plurality of diagnosis support processing results are obtained for one medical image. In some cases, the plurality of diagnosis support processing results are presented to the user, so that it is difficult for the user to check a desired diagnosis support processing result.

The present disclosure provides an information processing apparatus, a diagnosis support processing device, an information processing method, a diagnosis support processing method, an information processing program, and a diagnosis support processing program capable of providing a diagnosis support processing result that enables easy diagnosis. Solution to Problem

An information processing apparatus of a first aspect of the present disclosure comprises at least one processor, in which the processor is configured to perform diagnosis support processing on one medical image and derive a plurality of diagnosis support processing results, present selection information for allowing a user to select at least one diagnosis support processing result among the plurality of derived diagnosis support processing results, and output a diagnosis support processing result selected by the user according to the selection information.

According to an information processing apparatus of a second aspect of the present disclosure, in the information processing apparatus of the first aspect, the processor may be configured to output each diagnosis support processing result in association with the one medical image as a diagnosis support processing result.

According to an information processing apparatus of a third aspect of the present disclosure, in the information processing apparatus of the first aspect or the second aspect, the processor may be configured to present, as the selection information, information indicating an acquisition source for acquiring a diagnosis support processing result for each of the plurality of diagnosis support processing results.

According to an information processing apparatus of a fourth aspect of the present disclosure, in the information processing apparatus of any one of the first to third aspects, the processor may be configured to perform the diagnosis support processing for each part included in the one medical image and associate the part with a diagnosis support processing result, present information for selecting the part as the selection information, and output a diagnosis support processing result associated with the part selected by the user according to the selection information.

According to an information processing apparatus of a fifth aspect of the present disclosure, in the information processing apparatus of any one of the first to third aspects, the processor may be configured to perform the diagnosis support processing on the one medical image for each type of disease and associate the type of the disease with a diagnosis support processing result, present information for selecting the type of the disease as the selection information, and output a diagnosis support processing result associated with the type of the disease selected by the user according to the selection information.

A diagnosis support processing device of a sixth aspect of the present disclosure comprises at least one processor, in which the processor is configured to derive a plurality of diagnosis support processing results for one medical image, output each diagnosis support processing result in association with the one medical image, and output selection information for allowing a user to select at least one diagnosis support processing result among the plurality of derived diagnosis support processing results in association with the one medical image.

An information processing method of a seventh aspect of the present disclosure is a method executed by a computer, the method comprising performing diagnosis support processing on one medical image and deriving a plurality of diagnosis support processing results, presenting selection information for allowing a user to select at least one diagnosis support processing result among the plurality of derived diagnosis support processing results, and outputting a diagnosis support processing result selected by the user according to the selection information.

A diagnosis support processing method of an eighth aspect of the present disclosure is a method executed by a computer, the method comprising deriving a plurality of diagnosis support processing results for one medical image, outputting each diagnosis support processing result in association with the one medical image, and outputting selection information for allowing a user to select at least one diagnosis support processing result among the plurality of derived diagnosis support processing results in association with the one medical image.

An information processing program of a ninth aspect is a program causing a computer to execute a process comprising performing diagnosis support processing on one medical image and deriving a plurality of diagnosis support processing results, presenting selection information for allowing a user to select at least one diagnosis support processing result among the plurality of derived diagnosis support processing results, and outputting a diagnosis support processing result selected by the user according to the selection information.

In addition, a diagnosis support processing program of a tenth aspect of the present disclosure is a program causing a computer to execute a process comprising deriving a plurality of diagnosis support processing results for one medical image, outputting each diagnosis support processing result in association with the one medical image, and outputting selection information for allowing a user to select at least one diagnosis support processing result among the plurality of derived diagnosis support processing results in association with the one medical image.

### Advantageous Effects of Invention

According to the above-described aspects, the information processing apparatus, the diagnosis support processing device, the information processing method, the diagnosis support processing method, the information processing program, and the diagnosis support processing program of the present disclosure can provide a diagnosis support processing result that enables easy diagnosis.

### BRIEF DESCRIPTION OF THE DRAWINGS

Fig. 1 is a configuration diagram illustrating an example of an overall configuration of a mobile radiography apparatus of an exemplary embodiment.
Fig. 2 is a block diagram illustrating an example of the configuration of the mobile radiography apparatus of the exemplary embodiment.
Fig. 3 is a functional block diagram illustrating an example of a configuration related to a function of executing diagnosis support processing in a console of the exemplary embodiment.
Fig. 4 illustrates diagrams of examples of diagnosis support processing results obtained in a case in which a diagnosis support processing execution unit applies chest CAD to a radiation image and performs diagnosis support processing.
Fig. 5 is a flowchart illustrating an example of the flow of information processing by a console of the exemplary embodiment.
Fig. 6 is a diagram illustrating an example of a state in which acquisition source information and a radiation image acquired from a radiation detector are displayed on a display unit.
Fig. 7 is a diagram illustrating another example of a state in which the acquisition source information and the radiation image acquired from the radiation detector are displayed on the display unit.
Fig. 8 is a flowchart illustrating another example of the flow of the information processing by the console of the exemplary embodiment.
Fig. 9 is a diagram illustrating yet another example of a state in which the acquisition source information and the radiation image acquired from the radiation detector are displayed on the display unit.

### DESCRIPTION OF EMBODIMENTS

Hereinafter, exemplary embodiments of the present disclosure will be described in detail with reference to the drawings. In the following exemplary embodiment, a form will be described in which a radiation image is applied as an example of a medical image of the present disclosure and a console of a mobile radiography apparatus is applied as an example of an information processing apparatus according to the embodiment of the present disclosure. The present exemplary embodiments do not limit the present disclosure.

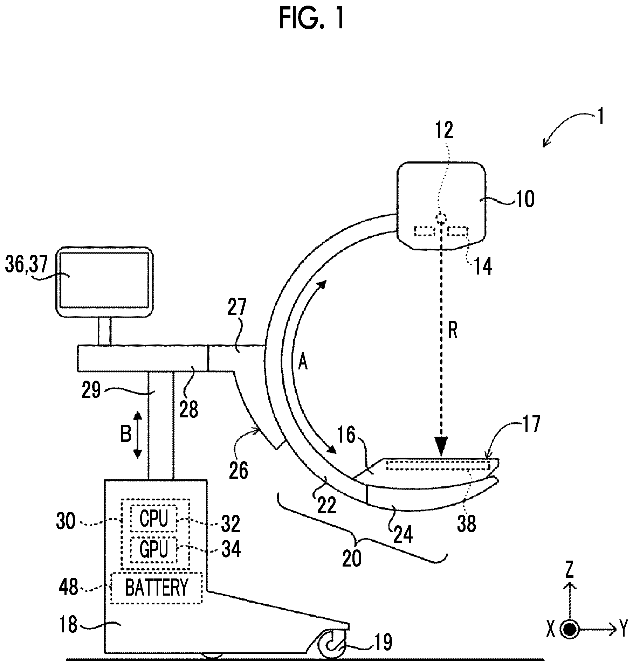

First, an example of an overall configuration of a mobile radiography apparatus according to the present exemplary embodiment will be described. Fig. 1 is a configuration diagram illustrating an example of the overall configuration of a mobile radiography apparatus 1 of the present exemplary embodiment.

As illustrated in Fig. 1, the mobile radiography apparatus 1 according to the present exemplary embodiment comprises a C-arm 20 having an arm portion 22 and a holding portion 24. A radiation emitting unit 10 that emits radiation R generated by a radiation source 12 is provided at one end of the arm portion 22.

The radiation source 12 and an irradiation field limiter 14 are stored in the radiation emitting unit 10. The radiation source 12 has a radiation tube (not illustrated) that generates radiation R and has a function of emitting the radiation R generated by the radiation tube. The irradiation field limiter 14 is a so-called collimator that has a function of limiting an irradiation field of the radiation R generated by the radiation tube. The irradiation field limiter 14 has, for example, a configuration in which four shield plates that is formed of lead or the like and shield the radiation R are disposed on respective sides of a quadrangle, and a quadrangular opening portion through which the radiation R is transmitted is formed in a center portion. The irradiation field limiter 14 changes the position of each shielding plate to change the size of the opening portion, thereby changing the irradiation field of the radiation R.

On the other hand, the holding portion 24 is provided at the other end of the arm portion 22. The holding portion 24 holds a housing portion 16. The housing portion 16 houses a radiation detector 38 that detects the radiation R to generate image data indicating a radiation image. The C-arm 20 according to the present exemplary embodiment has a function of changing an angle of the radiation detector 38 with respect to a Z direction (vertical direction) illustrated in Fig. 1.

The radiation detector 38 detects the radiation R transmitted through a subject. Specifically, the radiation detector 38 detects the radiation R that has entered into the housing portion 16 and that has reached a detection surface of the radiation detector 38, generates a radiation image based on the detected radiation R, and outputs image data indicating the generated radiation image. In the following, a series of operations of emitting the radiation R from the radiation source 12 and generating a radiation image by the radiation detector 38 may be referred to as "imaging". The type of the radiation detector 38 of the present exemplary embodiment is not particularly limited, and may be, for example, an indirect conversion type radiation detector that converts radiation R into light and converts the converted light into electric charge, or a direct conversion type radiation detector that converts radiation R into electric charge directly. In addition, the radiation detector 38 can capture at least one of a still image or a moving image.

An imaging surface 17 irradiated with the radiation R emitted from the radiation emitting unit 10 is provided on a side of the housing portion 16 which faces the radiation emitting unit 10. In the mobile radiography apparatus 1 of the present exemplary embodiment, a so-called source image distance (SID), which is a distance between the imaging surface 17 and the radiation source 12 of the radiation emitting unit 10 is a fixed value.

The C-arm 20 is held by a C-arm holding portion 26 to be movable in a direction of an arrow A illustrated in Fig. 1. In addition, the C-arm holding portion 26 has a shaft portion 27, and the shaft portion 27 connects the C-arm 20 to a bearing 28. The C-arm 20 is rotatable about the shaft portion 27 as a rotation axis.

In addition, as illustrated in Fig. 1, the mobile radiography apparatus 1 of the present exemplary embodiment comprises a main body portion 18 that is provided with a plurality of wheels 19 at the bottom portion. A support shaft 29 that is expanded and contracted in a Z-axis direction of Fig. 1 is provided on an upper portion side of a housing of the main body portion 18 in Fig. 1. The bearing 28 is held in an upper portion of the support shaft 29 to be movable in a direction of an arrow B.

In addition, a display unit 36 and an operating unit 37 are provided in the upper portion of the main body portion 18. The display unit 36 and the operating unit 37 function as a user interface. The display unit 36 provides an operator, such as a technician or a doctor, who performs capturing of a radiation image with the mobile radiography apparatus 1, with the captured radiation image or information related to capturing of the radiation image. The display unit 36 is not particularly limited, and examples thereof include a liquid crystal monitor, and a light emission diode (LED) monitor. In the present exemplary embodiment, a touch panel display integrated with the operating unit 37 is applied as an example of the display unit 36. In addition, the operating unit 37 is operated by an operator when giving an instruction related to capturing of the radiation image. The operating unit 37 is not particularly limited, and examples thereof include various switches, a touch panel, a touch pen, and a mouse. In addition, a plurality of operating units 37 may be provided. For example, a touch panel and a foot switch that is operated by the operator's feet may be provided as the operating unit 37. The operator of the present exemplary embodiment is an example of a user of the present disclosure.

In addition, a central processing unit (CPU) 32 and a graphics processing unit (GPU) 34 of a console 30 and a battery 48 that supplies power to each unit of the mobile radiography apparatus 1 are stored inside the main body portion 18.

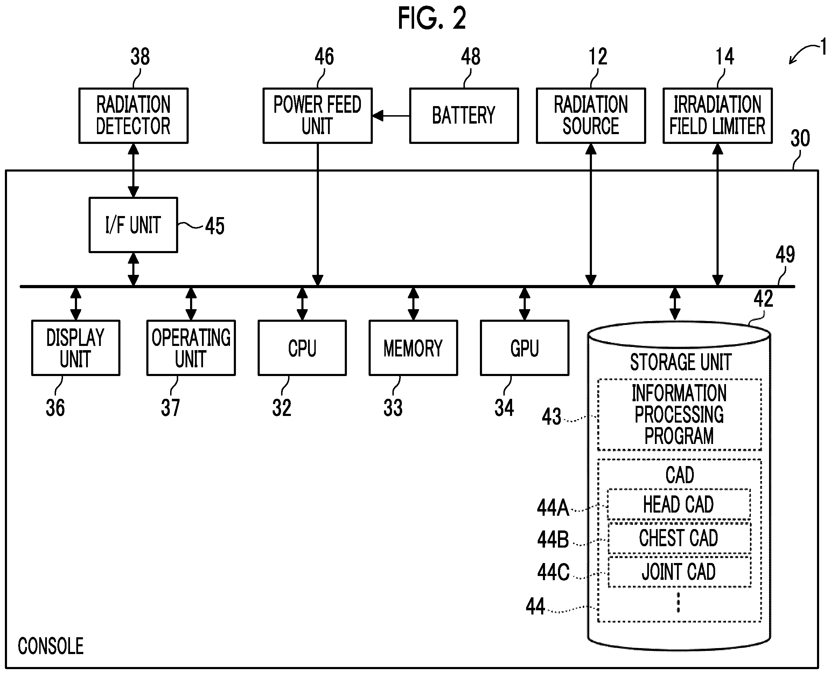

Fig. 2 is a block diagram illustrating an example of a configuration of the mobile radiography apparatus 1 of the present exemplary embodiment. As illustrated in Fig. 2, the mobile radiography apparatus 1 of the present exemplary embodiment comprises the radiation source 12, the irradiation field limiter 14, the console 30, the radiation detector 38, a power feed unit 46, and the battery 48.

The console 30 has a function of performing control related to capturing of the radiation image by the mobile radiography apparatus 1. The console 30 of the present exemplary embodiment is an example of the information processing apparatus of the embodiment of the present disclosure.

The console 30 comprises the CPU 32, a memory 33, the GPU 34, the display unit 36, the operating unit 37, a storage unit 42, and an I/F unit 45. The CPU 32, the memory 33, the GPU 34, the display unit 36, the operating unit 37, the storage unit 42, and the I/F unit 45 are connected to each other through a bus 49, such as a system bus or a control bus, such that various types of information can be exchanged therebetween. The radiation source 12, the irradiation field limiter 14, and the power feed unit 46 are also connected to the bus 49.

The CPU 32 reads out various programs, which include an information processing program 43 stored in the storage unit 42, to the memory 33 and performs processing corresponding to the program read out. Accordingly, the CPU 32 controls the operation of each unit of the mobile radiography apparatus 1. The CPU 32 of the present exemplary embodiment is an example of a processor of the present disclosure. The memory 33 is a work memory that is used to execute processing by the CPU 32. The GPU 34 has a function of applying a computer-assisted detection/diagnosis (CAD) 44 stored in the storage unit 42 to execute diagnosis support processing, which will be described in detail below, under the control of the CPU 32. The GPU 34 of the present exemplary embodiment is an example of a diagnosis support processing device of the embodiment of the present disclosure.

The storage unit 42 stores, for example, the information processing program 43, the CAD 44 which is an algorithm applied to the diagnosis support processing, the image data of the radiation image captured by the radiation detector 38, and various other types of information. The CAD 44 includes various algorithms corresponding to the types of diagnosis support processing which will be described below. For example, the CAD 44 of the present exemplary embodiment includes a plurality of types of CAD, such as a head CAD 44A, a chest CAD 44B, and a joint CAD 44C, which are applied to the radiation image in the diagnosis support processing. The algorithms included in the CAD 44 are not limited thereto. Specific examples of the storage unit 42 include a hard disk drive (HDD) and a solid state drive (SSD).

The I/F unit 45 communicates various types of information with the radiation detector 38 using wireless communication or wired communication. In addition, the I/F unit 45 performs communication of various types of information with an external device via a network using the wireless communication or the wired communication. Examples of the external device include a radiology information system (RIS) that manages an imaging order and picture archiving and communication system (PACS).

In addition, as described above, the power feed unit 46 is connected to the bus 49. The power feed unit 46 supplies electric power from the battery 48 to each unit of the mobile radiography apparatus 1. The power feed unit 46 includes a direct-current (DC)-DC converter that converts a direct-current voltage from the battery 48 to a voltage having a value according to a supply destination, a voltage stabilization circuit that stabilizes the value of the converted voltage, and the like. The battery 48 of the present exemplary embodiment is built in the main body portion 18 as described above. In this way, the mobile radiography apparatus 1 is driven by the battery 48 in a wireless manner. The mobile radiography apparatus 1 can charge the battery 48 or can operate with electric power from the commercial power supply by connecting a plug (not illustrated) of a power cord extending from a lower portion of the main body portion 18 to an outlet of the commercial power supply.

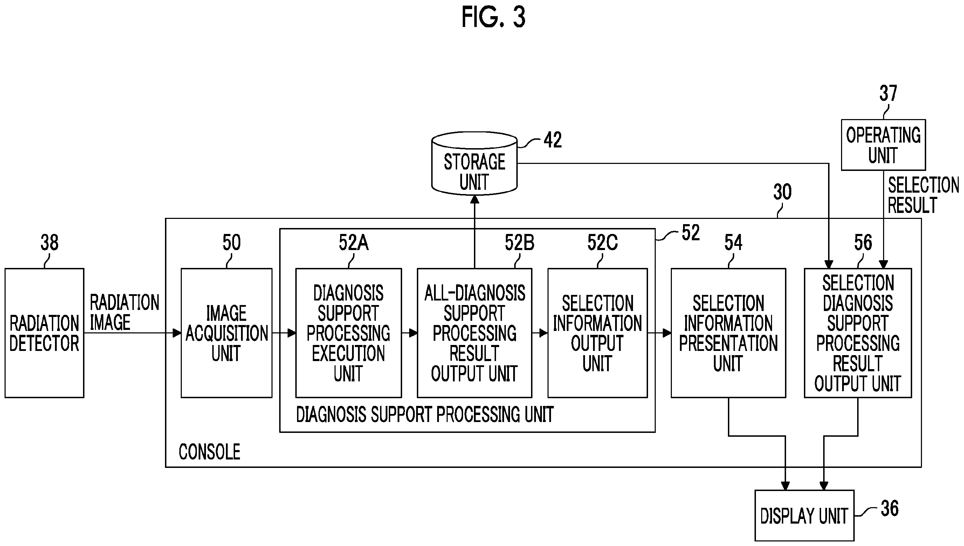

In addition, the console 30 of the present exemplary embodiment has a function of executing the diagnosis support processing for the radiation image captured by the radiation detector 38. Fig. 3 is a functional block diagram illustrating an example of a configuration related to the function of executing the diagnosis support processing in the console 30 of the present exemplary embodiment. As illustrated in Fig. 3, the console 30 comprises an image acquisition unit 50, a diagnosis support processing unit 52, a selection information presentation unit 54, and a selection diagnosis support processing result output unit 56. As an example, in the console 30 of the present exemplary embodiment, the CPU 32 executes the information processing program 43 stored in the storage unit 42, so that the CPU 32 functions as the image acquisition unit 50, the selection information presentation unit 54, and the selection diagnosis support processing result output unit 56, and the GPU 34 functions as the diagnosis support processing unit 52.

The image acquisition unit 50 has a function of acquiring the radiation image. Specifically, as described above, the image acquisition unit 50 of the present exemplary embodiment has a function of acquiring image data indicating the radiation image captured by the radiation detector 38. The image acquisition unit 50 outputs the acquired image data indicating the radiation image to the diagnosis support processing unit 52.

The diagnosis support processing unit 52 has a function of executing the diagnosis support processing for one radiation image and deriving a plurality of diagnosis support processing results. As illustrated in Fig. 3, the diagnosis support processing unit 52 of the present exemplary embodiment has a diagnosis support processing execution unit 52A, an all-diagnosis support processing result output unit 52B, and a selection information output unit 52C.

The diagnosis support processing execution unit 52Ahas a function of executing the diagnosis support processing for the radiation image input from the image acquisition unit 50 and deriving a plurality of diagnosis support processing results. Specifically, the diagnosis support processing execution unit 52A applies the algorithm of the CAD 44 stored in the storage unit 42 to the radiation image to perform the diagnosis support processing. In the present exemplary embodiment, the diagnosis support processing is executed for each part included in one radiation image to derive the diagnosis support processing result for each part, and associates the part with the diagnosis support processing result.

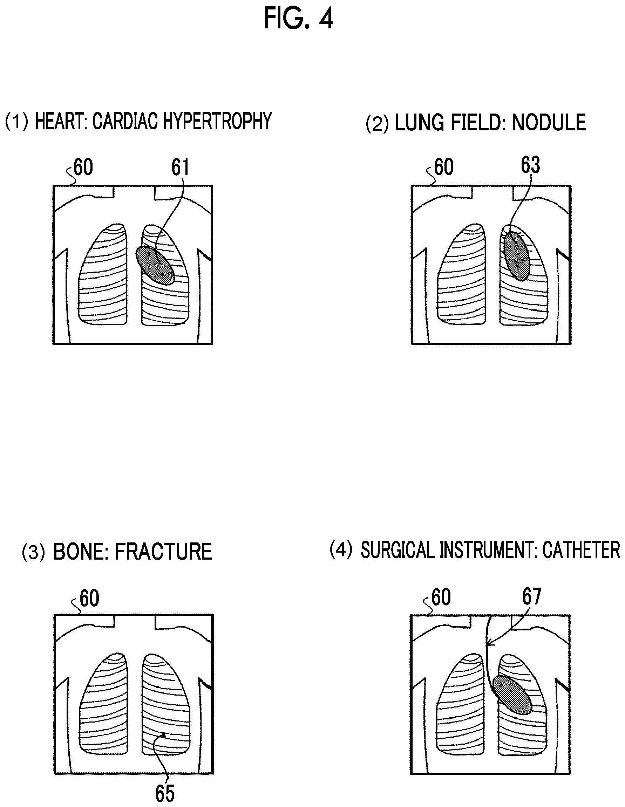

As an example, a case in which the diagnosis support processing execution unit 52A performs the diagnosis support processing for a radiation image obtained by imaging the chest of the subject will be described with reference to Fig. 4. In a case in which the diagnosis support processing execution unit 52A performs the diagnosis support processing for the radiation image obatined by imaging the chest, the diagnosis support processing execution unit 52A extracts the regions of the heart, the lung field, the bone, and the surgical instrument as each part of the chest from the radiation image. In the present embodiment, although the "surgical instrument" such as a catheter is also treated in the same manner as each part of the subject, regions other than the parts of the subject, such as the "surgical instrument", may be treated as the parts of the subject through which the surgical instrument passes. For example, in a case of the catheter, it may be treated as a part such as a blood vessel or a heart. The method by which the diagnosis support processing execution unit 52A extracts each part from the radiation image is not particularly limited. For example, the diagnosis support processing execution unit 52A may apply the existing image segmentation technology to the radiation image to extract each part from the radiation image.

Fig. 4 illustrates examples of the diagnosis support processing results obtained in a case in which the diagnosis support processing execution unit 52A applies the chest CAD 44B to the radiation image to perform the diagnosis support processing. (1) of Fig. 4 illustrates an example of a state in which the diagnosis support processing result of cardiac hypertrophy 61 is obtained by applying the chest CAD 44B to the heart. In addition, (2) of Fig. 4 illustrates an example of a state in which the diagnosis support processing result of a nodule 63 is obtained by applying the chest CAD 44B to the lung field. In addition, (3) of Fig. 4 illustrates an example of a state in which the diagnosis support processing result of fracture 65 of a rib is obtained by applying the chest CAD 44B to the bone. Furthermore, (4) of Fig. 4 illustrates an example of a state in which the diagnosis support processing result indicating a position of the catheter 67 is obtained by applying the chest CAD 44B to the surgical instrument.

The all-diagnosis support processing result output unit 52B outputs the diagnosis support processing result for each of all parts derived by the diagnosis support processing execution unit 52A by associating the part with the diagnosis support processing result to the storage unit 42. For example, as illustrated in (1) to (4) of Fig 4, the radiation image is output to the storage unit 42 in association with each part and the diagnosis support processing result. In the storage unit 42, the radiation image including a plurality of diagnosis support processing results are stored in the storage unit 42 in a state of being associated with the part. In this case, for each diagnosis support processing result, information indicating the stored location is generated as information indicating an acquisition source for acquiring the diagnosis support processing result. Hereinafter, information indicating the acquisition source for acquiring the diagnosis support processing result is referred to as "acquisition source information". Details of the acquisition source information will be described later.

The selection information output unit 52C has a function of outputting selection information for allowing an operator to select at least one diagnosis support processing result among the plurality of diagnosis support processing results derived by the diagnosis support processing execution unit 52A to the selection information presentation unit 54 in association with the radiation image. In the present exemplary embodiment, the above-described acquisition source information is applied as an example of the selection information.

The selection information presentation unit 54 has a function of presenting selection information for allowing the user to select at least one diagnosis support processing result among the plurality of diagnosis support processing results derived by the diagnosis support processing execution unit 52A. As an example, the selection information presentation unit 54 of the present exemplary embodiment displays the acquisition source information on a display unit 36 as will be described in detail below. The operator selects a desired diagnosis support processing result from the acquisition source information displayed on the display unit 36 using the operating unit 37.

The selection diagnosis support processing result output unit 56 has a function of outputting a diagnosis support processing result selected by the user according to the acquisition source information. Specifically, the selection diagnosis support processing result output unit 56 acquires the diagnosis support processing result from the storage unit 42 based on the acquisition source selected using the operating unit 37, and displays the acquired diagnosis support processing result on the display unit 36.

Since the selection diagnosis support processing result output unit 56 outputs each diagnosis support processing result in association with one radiation image as the diagnosis support processing result, one diagnosis support processing result desired by the operator is illustrated in one radiation image displayed on the display unit 36. Therefore, in a case in which the plurality of diagnosis support processing results are output, the number of radiation images corresponding to the number of diagnosis support processing results is displayed on the display unit 36.

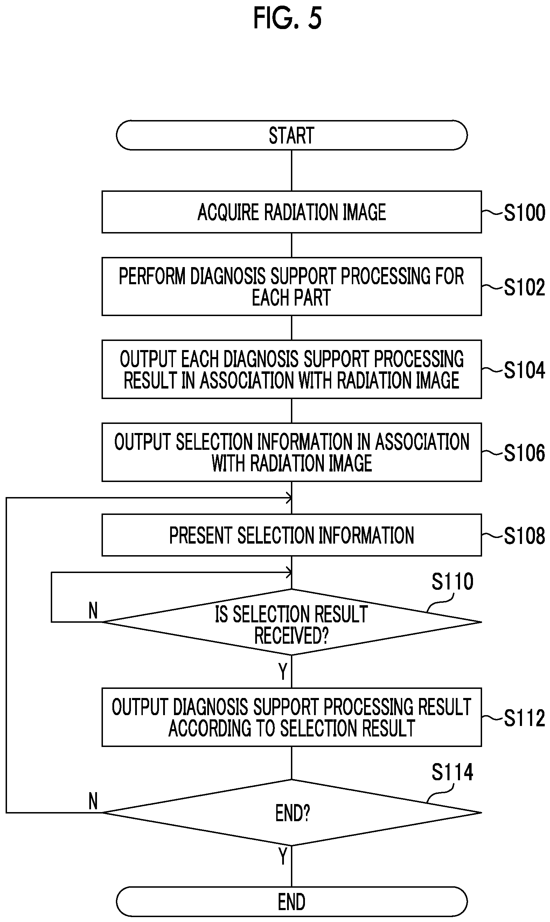

Next, an action of the console 30 related to the diagnosis support processing will be described with reference to the drawings. In a case in which an instruction to capture a radiation image corresponding to an imaging order is given, the console 30 of the present exemplary embodiment performs the information processing illustrated in Fig. 5. Fig. 5 is a flowchart illustrating an example of the flow of the information processing performed in the console 30 of the present exemplary embodiment. As an example, in the console 30 of the present exemplary embodiment, in a case in which an instruction to perform the diagnosis support processing given by the operator through the operating unit 37 is received, the CPU 32 executes the information processing program 43 stored in the storage unit 42 to execute the information processing illustrated in Fig. 5 as an example.

In step S100 of Fig. 5, the image acquisition unit 50 acquires a radiation image from the radiation detector 38.

In next step S102, the diagnosis support processing execution unit 52A performs the diagnosis support processing for each part for the radiation image acquired in Step S 100. Specifically, as described above, the diagnosis support processing execution unit 52A performs the diagnosis support processing for each part by extracting a part included in the radiation image and applying the algorithm of the CAD 44 stored in the storage unit 42 to each extracted part.

In next step S104, the all-diagnosis support processing result output unit 52B outputs each diagnosis support processing result derived in step S102 in association with the radiation image to the storage unit 42. Specifically, as described above, the all-diagnosis support processing result output unit 52B outputs the diagnosis support processing result for each of all parts by associating the part and the diagnosis support processing result with the radiation image to the storage unit 42.

In next step S106, the selection information output unit 52C outputs selection information for the operator to select the diagnosis support processing result to the selection information presentation unit 54 in association with the radiation image. Specifically, as described above, the selection information output unit 52C outputs the acquisition source information indicating the acquisition source of the diagnosis support processing result stored in the storage unit 42 to the selection information presentation unit 54, as the selection information.

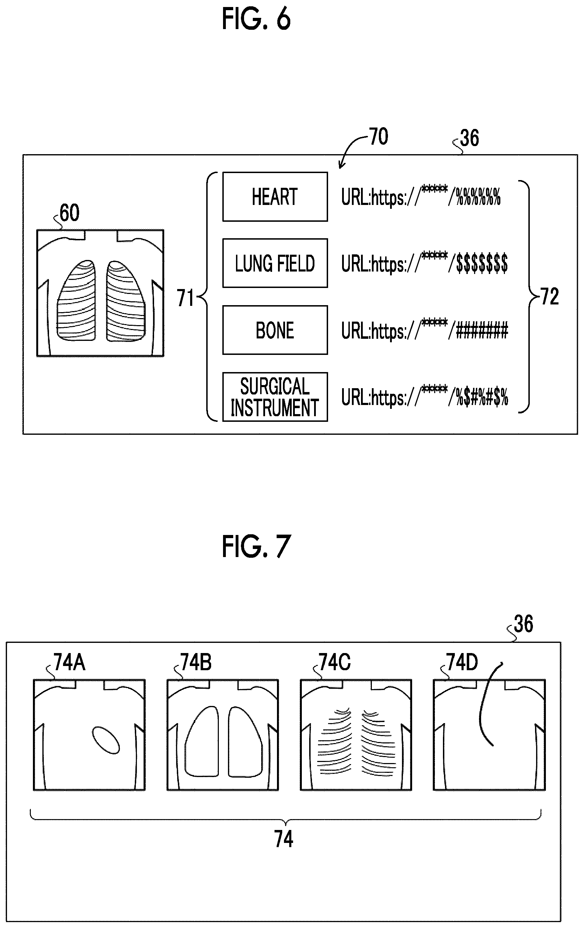

In next step S108, the selection information presentation unit 54 presents the selection information output in step S106 to the operator. As described above, the selection information presentation unit 54 presents the acquisition source information to the operator by displaying it on the display unit 36 as the selection information for allowing the user to select at least one diagnosis support processing result among the plurality of diagnosis support processing results derived in step S102. Fig. 6 is a diagram illustrating an example of a state in which acquisition source information 70 and a radiation image 60 acquired from the radiation detector 38 are displayed on the display unit 36. The acquisition source information 70 illustrated in Fig. 6 includes part information 71 indicating each part and uniform resource locator (URL) information 72 indicating an acquisition source of the diagnosis support processing result for each part. The operator selects a URL of the acquisition source according to the desired diagnosis support processing result from the URL information 72 displayed on the display unit 36, using the operating unit 37.

Therefore, in next step S110, the selection diagnosis support processing result output unit 56 determines whether or not the selection result selected by the operator is received. In the example illustrated in Fig. 6, it is determined whether or not any selection result of any of the URL information 72 is received. The determination in step S110 is a negative determination until the selection result is received. On the other hand, in a case in which the selection result is received, the determination in step S110 is an affirmative determination, and the processing proceeds to step S112.

In Step S112, the selection diagnosis support processing result output unit 56 outputs the diagnosis support processing result according to the received selection result to the display unit 36. In the example illustrated in Fig. 6, the diagnosis support processing result is acquired from the storage unit 42 based on the URL selected by the user from the URL information 72, and the acquired diagnosis support processing result is displayed on the display unit 36. For example, in a case in which the URL information 72 associated with a part of the heart is selected by the operator, the selection diagnosis support processing result output unit 56 displays, on the display unit 36, the radiation image 60 in which the cardiac hypertrophy 61 is obtained as the diagnosis support processing result and which is illustrated in (1) of Fig. 4. In addition, for example, in a case in which the URL information 72 associated with a part of the lung field is selected by the operator, the selection diagnosis support processing result output unit 56 displays, on the display unit 36, the radiation image 60 in which the nodule 63 is obtained as the diagnosis support processing result and which is illustrated in (2) of Fig. 4. In addition, for example, in a case in which the URL information 72 associated with a part of the bone is selected by the operator, the selection diagnosis support processing result output unit 56 displays, on the display unit 36, the radiation image 60 in which the fracture 65 is obtained as the diagnosis support processing result and which is illustrated in (3) of Fig. 4. Furthermore, for example, in a case in which the URL information 72 associated with a part of the surgical instrument is selected by the operator, the selection diagnosis support processing result output unit 56 displays, on the display unit 36, the radiation image 60 in which the catheter 67 is obtained as the diagnosis support processing result and which is illustrated in (4) of Fig. 4.

In addition, it may be possible for the operator to select a plurality of diagnosis support processing results. In this case, for example, in a case in which the operator selects the URL information 72 associated with a part of the heart and a part of the lung field, the selection diagnosis support processing result output unit 56 causes the display unit 36 to display the radiation images 60 illustrated in (1) and (2) of Fig. 4 side by side or switchably.

In next step S114, the selection diagnosis support processing result output unit 56 determines whether or not to end the information processing illustrated in Fig. 5. In the present exemplary embodiment, as a condition for ending the information processing, a case in which an end instruction given by the operator using the operating unit 37 is received is used. Until the end condition is satisfied, the determination in step S114 is a negative determination, the processing returns to step S108, and the selection diagnosis support processing result output unit 56 repeats the processing of steps S108 to S112. On the other hand, in a case in which the end condition is satisfied, the determination in step S114 is an affirmative determination, and the information processing illustrated in Fig. 5 ends.

As described above, in the console 30 of the mobile radiography apparatus 1 of the above-described embodiment, the GPU 34 performs the diagnosis support processing for one radiation image and derives a plurality of diagnosis support processing results. In addition, the CPU 32 presents selection information for allowing the operator to select at least one diagnosis support processing result among the plurality of derived diagnosis support processing results and outputs the diagnosis support processing result selected by the operator according to the selection information.

In addition, the GPU 34 of the above-described embodiment derives the plurality of diagnosis support processing results for one radiation image, outputs one radiation image in association with each diagnosis support processing result, and outputs selection information for allowing the operator to select at least one diagnosis support processing result among the plurality of derived diagnosis support processing results in association with the one radiation image.

Unlike the above-described embodiment, in a case in which the diagnosis support processing result that is not selected by the operator for one radiation image, that is, the diagnosis support processing result that is not desired by the operator is output, the diagnosis support processing result is provided to the operator in a state in which the plurality of diagnosis support processing results are superimposed. For example, the diagnosis support processing results are displayed on the display unit 36 in a state in which (1) to (4) Fig. 4 are superimposed. Since the diagnosis support processing results are superimposed, it may be difficult for the operator to check the desired diagnosis support processing result. In contrast, according to the above-described embodiment, the diagnosis support processing result selected by the operator among the plurality of diagnosis support processing results obtained for one radiation image is output to the operator. Since the diagnosis support processing result that is not selected by the operator is not output, for example, it is suppressed that the diagnosis support processing result is displayed in a superimposed manner. Therefore, according to the above-described embodiment, it is possible to provide a diagnosis support processing result that enables easy diagnosis. In addition, according to the above-described embodiment, since the diagnosis support processing result that enables easy diagnosis is provided, it is possible to improve the diagnosis accuracy.

In the above-described embodiment, although the form is described in which the URL information 72, which is the acquisition source information, is presented as the selection information for the operator to select the diagnosis support processing result as illustrated in Fig. 6, the selection information is not limited to the embodiment. For example, instead of the URL information 72 indicating the URL itself or together with the URL itself, a link to the URL may be presented as the selection information. For example, in the form illustrated in Fig. 6, the part information 71 may also serve as a link to the URL. In addition, for example, an image indicating the diagnosis support processing result, in other words, an image indicating the diagnosis support processing result stored in the selected URL may be presented. In addition, for example, as the example illustrated in Fig. 7, information for selecting each part associated with the diagnosis support processing result may be provided as the selection information 74. In the example illustrated in Fig. 7, selection information 74A indicating the heart, selection information 74B indicating the lung field, selection information 74C indicating the bone, and selection information 74D indicating the surgical instrument are included. For example, a segmentation image obtained in a case in which the diagnosis support processing execution unit 52A extracts each part from the radiation image may be applied to the selection information 74A to 74D which are images indicating each of these parts.

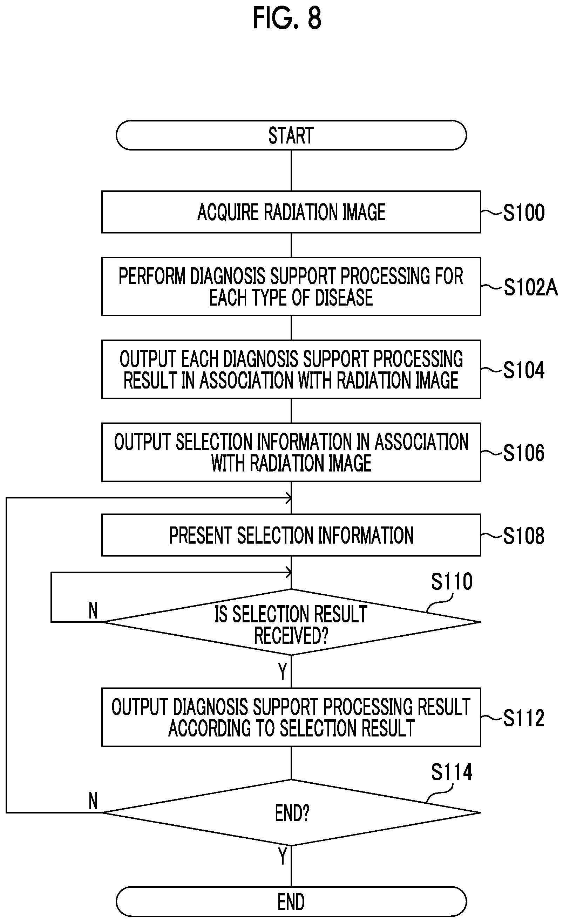

In addition, in the above-described embodiment, although the form in which the diagnosis support processing unit 52 performs the diagnosis support processing for each part has been described, the form in which the diagnosis support processing unit 52 performs the diagnosis support processing is not limited to this embodiment. For example, the diagnosis support processing execution unit 52A of the diagnosis support processing unit 52 may perform the diagnosis support processing for each type of disease. In the case of the embodiment, for example, in each processing of the diagnosis support processing unit 52 described above, each part may be replaced with each type of disease. For example, in the information processing performed in the console 30, as illustrated in Fig. 8, the processing of step S102Ais performed instead of step S 102 of the above-described information processing (refer to Fig. 5). In Step S 102A, the diagnosis support processing execution unit 52A performs the diagnosis support processing for each type of disease for the radiation image. For example, in the case of a radiation image obtained by imaging the chest of the subject, the diagnosis support processing is performed for each type of disease, such as "heart disease", "nodule", "pneumothorax", "fracture", and "surgical instrument". In this case, instead of the CAD for each part, such as the head CAD 44A, the CAD for each type of disease may be applied as the CAD 44 used in the diagnosis support processing.

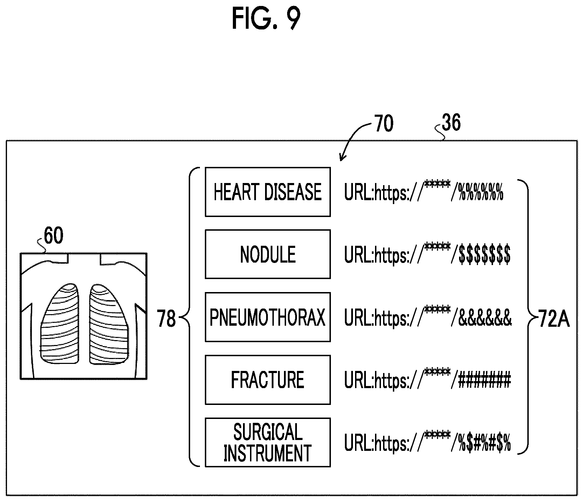

In addition, Fig. 9 illustrates an example of the form in which the selection information presentation unit 54 presents the acquisition source information 70, which is the selection information, to the operator by displaying it on the display unit 36 in step S108 of the information processing of the embodiment. The acquisition source information 70 illustrated in Fig. 9 includes disease information 78 indicating the type of each disease and URL information 72A indicating an acquisition source of the diagnosis support processing result for each disease type. The operator selects the URL of the acquisition source according to the desired diagnosis support processing result from the URL information 72A displayed on the display unit 36, using the operating unit 37. Accordingly, the diagnosis support processing result according to the type of disease is provided to the operator.

In addition, in the above-described embodiment, although the form in which the diagnosis support processing result is stored in the storage unit 42 has been described, the storage destination of the diagnosis support processing result is not limited to the storage unit 42. For example, a storage unit different from the storage unit 42 in the mobile radiography apparatus 1 may be used, or a storage device outside the mobile radiography apparatus 1 or the like may be used. In a case in which the storage device outside the mobile radiography apparatus 1 is used, the plurality of diagnosis support processing results may be stored in the PACS as a multi-frame format of digital imaging and communications in medicine (DICOM).

In addition, in the above-described embodiment, although the form in which the diagnosis support processing unit 52 applies the CAD algorithm to perform the diagnosis support processing has been described, the configuration to perform the diagnosis support processing or the CAD is not limited to the embodiment. For example, the diagnosis support processing unit 52 may apply artificial intelligence (AI) technology to perform the diagnosis support processing or may apply a trained model, which has been subjected to machine learning by deep learning or the like, to perform the diagnosis support processing.

In addition, in the above-described embodiment, although the form in which the console 30 is an example of the information processing apparatus according to the embodiment of the present disclosure has been described, an apparatus other than the console 30 may comprise the function of the information processing apparatus according to the embodiment of the present disclosure. In other words, an apparatus other than the console 30 may comprise some or all of the image acquisition unit 50, the diagnosis support processing unit 52, the selection information presentation unit 54, and selection diagnosis support processing result output unit 56. For example, the CPU 32 that functions as the console 30 and the GPU 34 and the storage unit 42 that function as the diagnosis support processing unit 52 may be separate entities, or the GPU 34 may be provided as a so-called GPU box.

In addition, in the above-described embodiment, although the form in which the mobile radiography apparatus having the C-arm is applied as an example of the medical imaging apparatus that captures a radiation image has been described, the medical imaging apparatus is not limited to the embodiment. For example, the medical imaging apparatus may be configured to use a mobile cart having the radiation emitting unit 10 and the radiation detector 38, which is a so-called electronic cassette, in combination. In addition, for example, it may be a portable medical imaging apparatus that is carried and moved by the operator. In addition, the medical imaging apparatus is not limited to the mobile medical imaging apparatus and may be a stationary medical imaging apparatus. In addition, for example, the imaging apparatus may be a medical imaging apparatus that captures a computed tomography (CT) image, an ultrasound image, or the like.

In addition, in the above-described embodiment, the following various processors can be used as the hardware structure of processing units that performs various types of processing, such as the image acquisition unit 50, the diagnosis support processing unit 52, the selection information presentation unit 54, and the selection diagnosis support processing result output unit 56. The various processors include a programmable logic device (PLD) that is a processor of which the circuit configuration can be changed after manufacture, such as a field programmable gate array (FPGA), a dedicated electrical circuit that is a processor having circuit configuration dedicatedly designed to perform specific processing, such as an application specific integrated circuit (ASIC), and the like in addition to a CPU that is a general-purpose processor functioning as various processing units by executing software (program) as described above.

One processing unit may be composed of one of the various processors or may be composed of a combination of two or more processors of the same type or different types (for example, a combination of a plurality of FPGAs or a combination of a CPU and an FPGA). In addition, a plurality of processing units may be formed of one processor.

As an example in which a plurality of processing units are formed of one processor, first, there is an aspect in which one processor is formed of a combination of one or more CPUs and software as typified by a computer, such as a client or a server, and functions as a plurality of processing units. Second, there is an aspect where a processor fulfilling the functions of the entire system, which includes a plurality of processing units, by one integrated circuit (IC) chip as typified by System On Chip (SoC) or the like is used. In this way, various processing units are formed using one or more of the above-described various processors as hardware structures.

Furthermore, more specifically, electrical circuitry in which circuit elements, such as semiconductor elements, are combined can be used as the hardware structures of these various processors.

In addition, an aspect in which the information processing program 43 is stored (installed) in the storage unit 42 in advance has been described in each of the above-mentioned embodiments, but the present disclosure is not limited thereto. The information processing program 43 may be provided in a form in which the information processing program 43 is recorded in recording mediums, such as a compact disc read only memory (CD-ROM), a digital versatile disc read only memory (DVD-ROM), and a universal serial bus (USB) memory. In addition, the information processing program 43 may be downloaded from an external device through a network.

The disclosure of JP 2021-080524, filed on May 11, 2021 is incorporated herein by reference in its entirety.

All documents, patent applications, and technical standards disclosed in the present specification are incorporated in the present specification by reference such that the incorporation of each of the documents, the patent applications, and the technical standards by reference is specific and is as detailed as each of the documents, the patent applications, and the technical standards.

## Claims

1. An information processing apparatus comprising:
at least one processor, the processor being configured to:
perform diagnosis support processing on one medical image and derive a plurality of diagnosis support processing results;
present selection information for allowing a user to select at least one diagnosis support processing result among the plurality of derived diagnosis support processing results; and
output a diagnosis support processing result selected by the user according to the selection information.

2. The information processing apparatus according to claim 1, wherein the processor is configured to output each diagnosis support processing result in association with the one medical image as a diagnosis support processing result.

3. The information processing apparatus according to claim 1, wherein the processor is configured to present, as the selection information, information indicating an acquisition source for acquiring a diagnosis support processing result for each of the plurality of diagnosis support processing results.

4. The information processing apparatus according to claim 1, wherein the processor is configured to:
perform the diagnosis support processing for each part included in the one medical image and associate the part with a diagnosis support processing result;
present information for selecting the part as the selection information; and
output a diagnosis support processing result associated with the part selected by the user according to the selection information.

5. The information processing apparatus according to claim 1, wherein the processor is configured to:
perform the diagnosis support processing on the one medical image for each type of disease and associate the type of the disease with a diagnosis support processing result;
present information for selecting the type of the disease as the selection information; and
output a diagnosis support processing result associated with the type of the disease selected by the user according to the selection information.

6. A diagnosis support processing device comprising:
at least one processor, the processor being configured to:
derive a plurality of diagnosis support processing results for one medical image;
output each diagnosis support processing result in association with the one medical image; and
output selection information for allowing a user to select at least one diagnosis support processing result among the plurality of derived diagnosis support processing results in association with the one medical image.

7. An information processing method executed by a computer, the method comprising:
performing diagnosis support processing on one medical image and deriving a plurality of diagnosis support processing results;
presenting selection information for allowing a user to select at least one diagnosis support processing result among the plurality of derived diagnosis support processing results; and
outputting a diagnosis support processing result selected by the user according to the selection information.

8. A diagnosis support processing method executed by a computer, the method comprising:
deriving a plurality of diagnosis support processing results for one medical image;
outputting each diagnosis support processing result in association with the one medical image; and
outputting selection information for allowing a user to select at least one diagnosis support processing result among the plurality of derived diagnosis support processing results in association with the one medical image.

9. An information processing program causing a computer to execute a process comprising:
performing diagnosis support processing on one medical image and deriving a plurality of diagnosis support processing results;
presenting selection information for allowing a user to select at least one diagnosis support processing result among the plurality of derived diagnosis support processing results; and
outputting a diagnosis support processing result selected by the user according to the selection information.

10. A diagnosis support processing program causing a computer to execute a process comprising:
deriving a plurality of diagnosis support processing results for one medical image;
outputting each diagnosis support processing result in association with the one medical image; and
outputting selection information for allowing a user to select at least one diagnosis support processing result among the plurality of derived diagnosis support processing results in association with the one medical image.
